# EUROPEAN PATENT APPLICATION

(11) **EP 3 210 606 A1**
(43) Date of publication of application: **30.08.2017**
(21) Application number: 17152965.4
(22) Date of filing: 06.09.2010
(51) Int. Cl.: A61K 31/4409, A61P 25/00

(54) **DURABLE TREATMENT WITH 4-AMINOPYRIDINE IN PATIENTS WITH DEMYELINATION**

(30) Priority: 04.09.2009 US 240100 P
(62) Divisional of application: 10814616.8
(71) Applicant: Acorda Therapeutics, Inc., Ardsley, NY 10502 (US)
(72) Inventor: WESSEL, Thomas, C., Lenox, MA Massachusetts 01240 (US); BLIGHT, Andrew, Mahopac, NY New York 10591 (US)
(74) Representative: Carpmaels & Ransford LLP

(57) **Abstract**

Disclosed herein are methods and compositions related to the durable use of aminopyridines, such as 4-aminopyridine, to improve impairments of patients with a demyelinating condition, such as MS.

## Description

This application claims priority of U.S. Provisional Patent application serial number 61/240,100 filed September 4, 2009, which is incorporated herein by reference in its entirety.

### FIELD OF THE INVENTION

The present invention relates to neurology and medicine. In a particular embodiments, the invention relates to the use of sustained release 4-aminopyridine to improve or stabilize patients that have demyelination of nerve cells, such as patients with Multiple sclerosis (MS).

### BACKGROUND OF THE INVENTION

MS is understood to be an autoimmune disease and is characterized by areas of demyelination (lesions) in the central nervous system (CNS). This characteristic demyelination and associated inflammatory response leads to abnormal impulse conduction or conduction block in nerve fibers traversing the lesions. Lesions can occur throughout the CNS but certain sites such as the optic nerve, brainstem, spinal cord, and periventricular regions seem particularly vulnerable. Impaired action potential conduction is probably the major contributor to the symptoms most often reported (e.g., paralysis, visual abnormalities, muscle weakness, nystagmus, sensory abnormalities, and speech disturbances). Other conditions, in addition to MS, are understood also to lead to demyelination, such as traumatic brain injury, spinal cord injury, etc..

With advances in the understanding of medical conditions, cognitive dysfunction has come to be seen as a significant issue for patients with MS. Controlled neuropsychological studies have shown that a substantial proportion of multiple sclerosis patients experience cognitive dysfunction. Recent memory, sustained attention, conceptual-abstract reasoning, and speed of information processing are impaired in about 50% of patients, whereas language functions are relatively spared. (Rao et al., Conference report: workshop on neurobehavioral disorders in multiple sclerosis. Arch Neurol 1993;50:658-662 Rao *et al.* demonstrated that a subgroup of MS patients with cognitive dysfunction were less likely to be employed, less likely to engage in social and recreational activities, and required greater personal assistance than a subgroup of cognitively intact patients with MS, despite both subgroups having an equivalent degree of physical disability. (Rao et al., Cognitive dysfunction in multiple sclerosis; Impact on employment and social functioning. Neurology 1991;41:692-696)

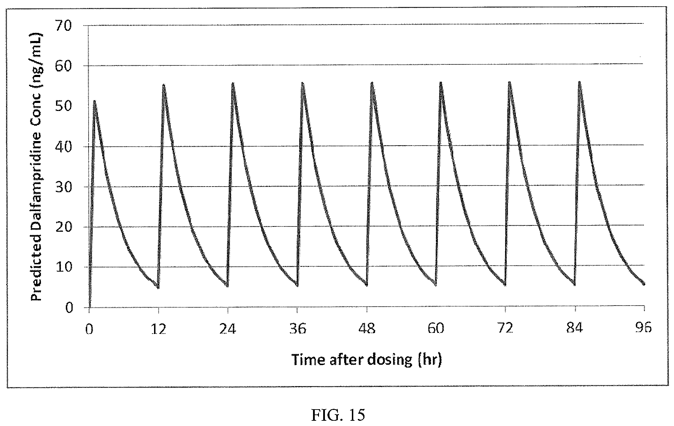

Studies of 4-Aminopyridine (4-aminopyridine) have been conducted using intravenous (i.v.) administration and immediate-release (IR) oral capsule formulations in addition to controlled-release or sustained-release formulations. Administration of IR capsules resulted in rapid and short-lasting peaks of 4-aminopyridine in the plasma (FIG. 15). Early pharmacokinetic studies were conducted using an immediate release (IR) formulation for oral administration, which consisted of 4-aminopyridine powder in a gelatin-based capsule or oral solution. Administration resulted in rapidly changing 4-aminopyridine plasma levels that were not well tolerated. A sustained-release matrix tablet (referred to herein as Fampridine-SR) was then developed. Fampridine-SR is available in the United States under the trade name Ampyra®, Acorda Therapeutics, Hawthorne, NY. The Fampridine-SR matrix tablet showed improved stability and an appropriate pharmacokinetic profile for twice-daily dosing.

Studies in people with multiple sclerosis (MS), including Phase 1, 2, and 3 clinical trials, indicate that the drug 4-aminopyridine improves a variety of neurological functions that are impaired by this disease, with particular attention focused on the effects of the drug to improve ambulation and leg strength.

There remains a need in the art for methods of ameliorating problems in patient populations subject to demyelinating or traumatic conditions.

### SUMMARY OF THE INVENTION

In the description, figures and tables herein, a number of terms are used. In order to provide a clear and consistent understanding of the specification and claims, the following definitions are provided:

**DEFINITIONS**

| **Abbreviation or Specialist Term** | **Explanation** |
|---|---|
| ADME | Absorption, distribution, metabolism, and excretion |
| Aₑ | Amount of drug excreted |
| APD₃₀, APD₅₀, APD₉₀ | Action potential duration 30%, 50%, 90% |
| AUC | Area under the concentration-time curve |
| AUC₍₀₋ₜ₎, AUC_{(0-∞)} or AUC_{(0-inf)} | Area under the plasma concentration *versus* time curve, to the last quantifiable level, and extrapolated to infinity |
| AUC₍₀₋₁₂₎, AUC₍₀₋₂₄₎ | Area under the plasma concentration *versus* time curve, 0-12 hours, 0-24 hours |
| b.i.d. (bid) | Twice daily |
| ¹⁴C | Radioactive carbon 14 |
| CHO | Chinese hamster ovary |
| CI | Confidence interval |
| CL/F | Apparent total body clearance after administration |
| Cl_{R} | Renal clearance |
| Cm | Centimeter |
| Cₘₐₓ | Maximum measured plasma concentration |
| CNS | Central nervous system |
| CR | Controlled-release |
| CrCl | Creatinine clearance |
| CumAₑ | Cumulative amount of drug excreted |
| CYP, CYP 450 | Cytochrome p450 isoenzymes |
| ECG | Electrocardiogram |
| EEG | Electroencephalogram |
| F | Female |
| FOB | Functional Observation Battery |
| 4-AP | 4-Aminopyridine, fampridine, dalfampridine |
| g, kg, mg, µg, ng | Gram, kilogram, milligram, microgram, nanogram |
| GABA | Gamma-aminobutyric acid |
| GLP | Good Laboratory Practice |
| h, hr | Hour |
| HDPE | High-density polyethylene |
| hERG | Human ether-à-go-go related gene |
| HPLC | High performance liquid chromatography |
| IC₅₀ | 50% Inhibitory concentration |
| I_{Kr} | Potassium ion channel whose activity is measured in the hERG assay |
| Improvement | Designates an alteration in a parameter in a desired direction. As used herein, "improvement" also comprises stabilization of a parameter that would otherwise be deteriorating or moving in a non-desired direction. |
| IND | Investigational New Drug application |
| IR | Immediate-release |
| i.v. (iv) | Intravenous |
| K⁺ | Potassium |
| Kₑₗ | Elimination constant |
| L, mL | Liter, milliliter |
| LCMS, LC/MS/MS | Liquid chromatography / mass spectrometry |
| LD₅₀ | Median lethal dose |
| Ln | Natural log |
| LOQ | Limit of quantitation |
| M | Male |
| Min | Minute |
| mM, µM | Millimolar, micromolar |
| MRT | Mean residence time |
| MS | Multiple sclerosis |
| MTD | Maximum tolerated dose |
| NA | Not applicable |
| ND | None detected |
| NDA | New Drug Application |
| NE | Not evaluable |
| NF | National Formulary |
| NOAEL | No observable adverse effect level |
| NOEL | No observable effect level |
| Norm | Normalized |
| NZ | New Zealand |
| pₐₚₚ | Apparent permeability coefficient |
| p.o. | Oral |
| SAE | Serious adverse event |
| SCI | Spinal cord injury |
| SD | Standard deviation |
| Sec | Second |
| SEM | Standard error of the mean |
| SPF | Specific pathogen-free |
| SR | Sustained-release |
| SS | Steady state |
| t_{½} | Apparent terminal elimination half-life |
| t.i.d. (tid) | Three times daily |
| TK | Toxicokinetics |
| TLC | Thin layer chromatography |
| Tₘₐₓ | Time of the maximum measured plasma concentration |
| USP | United States Pharmacopeia |
| UTI | Urinary tract infection |
| V_{d} | Volume of distribution |
| V_{dss} | Volume of distribution at steady state |

It is to be noted that when Fampridine-SR is used here, it is to be seen as merely exemplary of a sustained release 4-AP composition. Other sustained release compositions are within the scope of the invention. Moreover, when BID other uses of sustained release 4AP are disclosed, this is merely an example, as alternative dosing regimens (without limitation such as QD or TID) are within the scope of the present invention. Thus, alternative release formulations (other than sustained release) and alternative dosing periodicity (other than BID) are within the scope of the invention.

When used in conjunction with the word "comprising" or other open language in the claims, the words "a" and "an" denote "one or more."

Although the present invention has been described in considerable detail herein with reference to certain preferred embodiments thereof, other versions are possible. The spirit and scope of the appended claims should not be limited to the description and the preferred versions contain within this specification. All documents referred to herein are fully incorporated by reference herein for all purposes.

### BRIEF DESCRIPTION OF THE DRAWINGS

The following drawings form part of the present specification and are included to demonstrate certain aspects of the present disclosure in greater detail. The invention may be better understood by reference to one of these drawings in combination with the detailed description of specific embodiments presented herein.

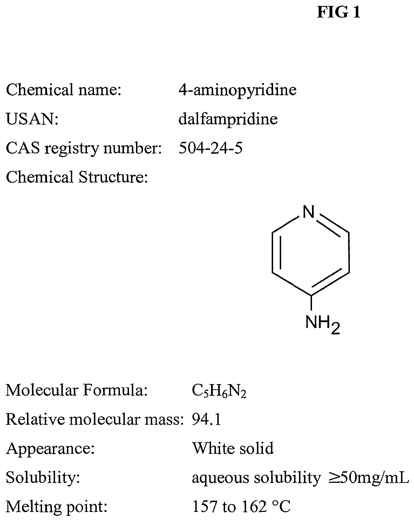

FIG. 1 shows information regarding 4-aminopyridine.
FIG. 2 MS-F203 primary endpoint: Fampridine-SR increases timed walk.

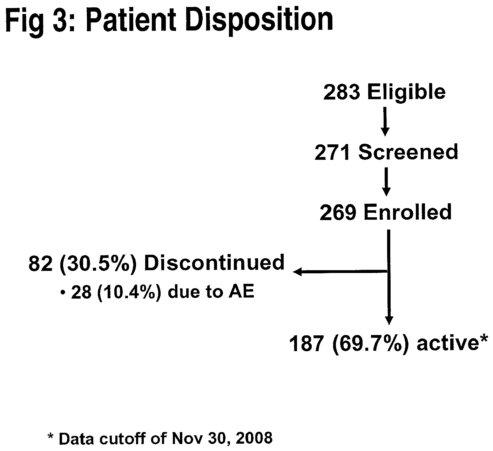

FIG. 3 Patient disposition.

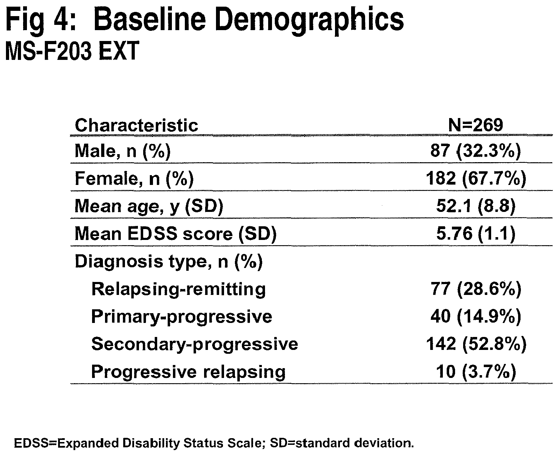

FIG. 4 Baseline demographics.

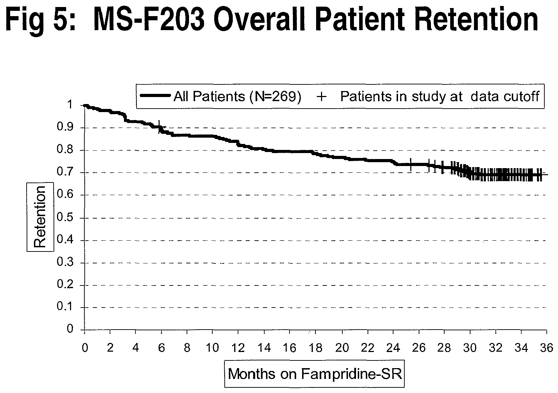

FIG. 5 MS-F203 overall patient retention.

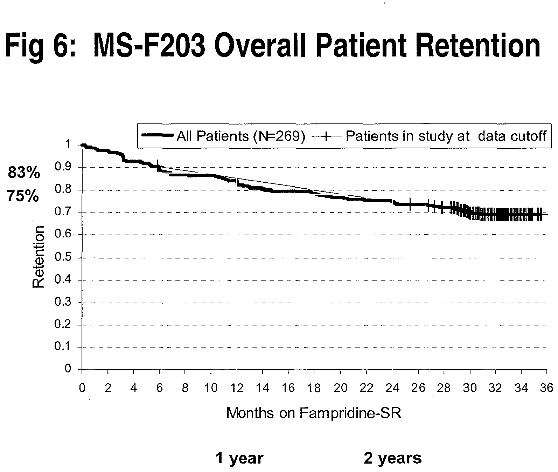

FIG. 6 MS-F203 overall patient retention.

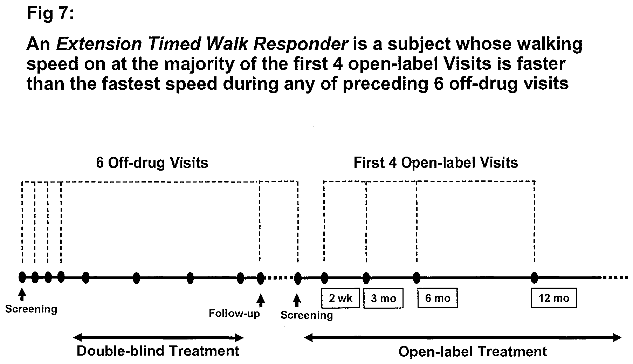

FIG. 7 Extension timed walk.

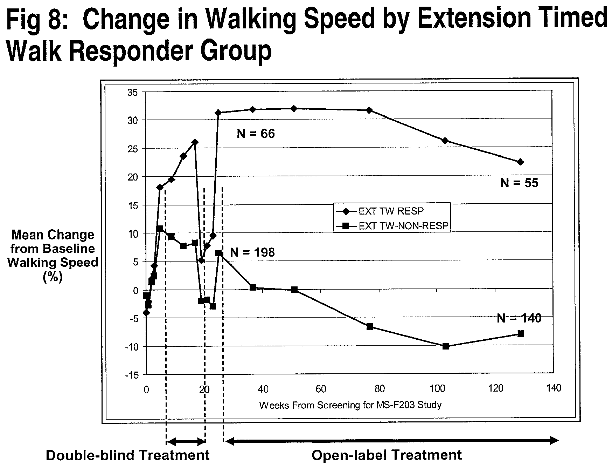

FIG. 8 Change in walking speed by extension timed walk responder group.

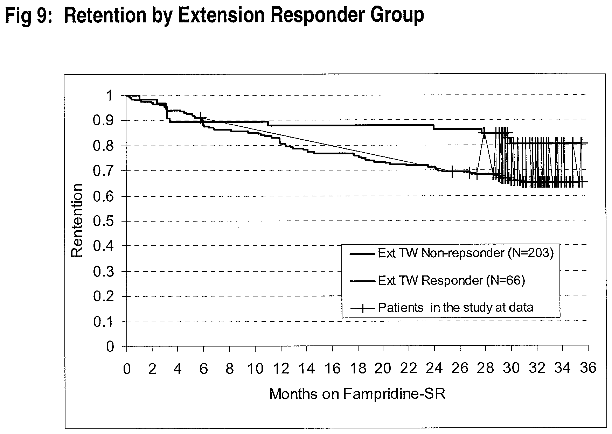

FIG. 9 Retention by extension responder group.

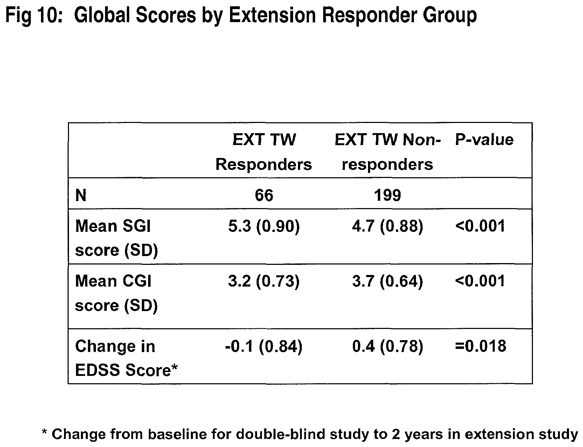

FIG. 10 Global scores by extension responder group.

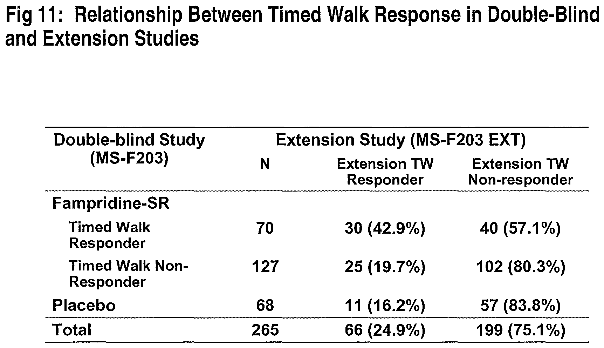

FIG. 11 Relationship between timed walk response in double-blind and extension studies.

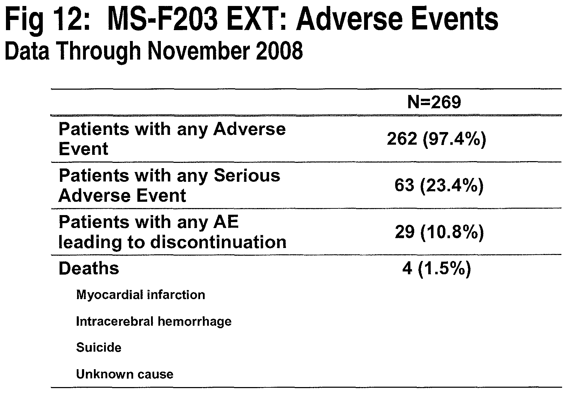

FIG. 12 MS F203 EXT: adverse events.

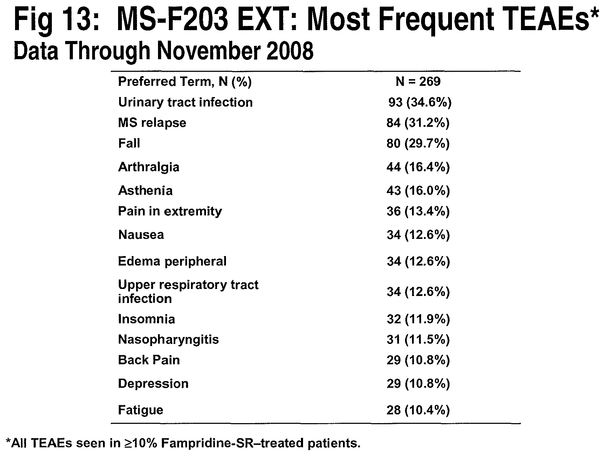

FIG. 13 MS-F203 EXT: most frequent Treatment-emergent adverse events (TEAEs).

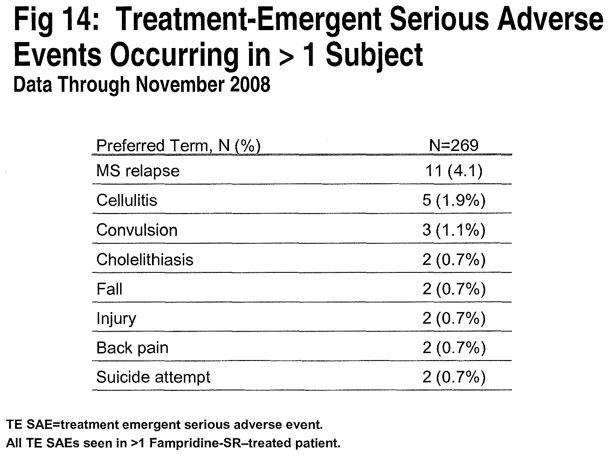

FIG. 14 Treatment-emergent serious adverse events occurring in >1 subject.
FIG. 15 4-aminopyridine in the plasma after administration of 10 mg IR fampridine.
FIG. 16 Steady state pharmacokinetic profile for sustained release 4-aminopyridine.

### DETAILED DESCRIPTION OF THE INVENTION

The chemical 4-aminopyridine is a potassium (K+) channel blocker approved or under evaluation as a treatment, *e.g*., for improving neurological and muscular functions in patients with Multiple Sclerosis (MS); fampridine is the name of this compound in the rest of the world. Dalfampridine is the United States Adopted Name (USAN) for 4-aminopyridine (4 AP), which has a molecular formula of C₅H₆N₂ and molecular weight of 94.1. "Fampridine," "dalfampridine," and "4-aminopyridine" will be used throughout this specification to refer to the active drug substance. 4-aminopyridine has been formulated as a sustained-release (SR) matrix tablet in various strengths from 5 to 40 mg. Fampridine-SR, is available in the United States, *e.g*., at a strength of 10 mg tablets under the trade name Ampyra®, Acorda Therapeutics, Hawthorne, NY. In one embodiment, the following excipients are generally included in each tablet: hydroxypropyl methylcellulose, USP; microcrystalline cellulose, USP; colloidal silicon dioxide, NF; magnesium stearate, USP; and Opadry White.

Effects on Axonal Conduction - Pharmacologically, the K+ channel blocking properties of 4-aminopyridine and its effects on action potential conduction in demyelinated nerve fiber preparations have been extensively characterized. At low concentrations that are relevant to clinical experience, in the range of 0.2 to 2 µM (18 to 180 ng/mL), 4-aminopyridine is able to block certain voltage-dependent K+ channels in neurons. It is this characteristic that appears to explain the ability of the drug to restore conduction of action potentials in demyelinated nerve fibers. At higher (millimolar) concentrations, 4-aminopyridine affects other types of K+ channels in both neural and non-neural tissues. Blockade of repolarizing K+ currents can increase synaptic transmission throughout the nervous system by increasing the duration of the pre-synaptic action potential. A range of neurological effects consistent with increased excitability of presynaptic nerve terminals occurs with clinically relevant doses of 4-aminopyridine.

The K+ channels blocked by low concentrations of 4-aminopyridine are partially responsible for repolarization of neuronal action potentials. These appear to include those found under the myelin sheath in myelinated nerve fibers of adult mammals. These channels are located primarily in the paranodal and internodal membrane of the axon (Waxman and Ritchie, 1993) where they are not significantly activated by the passage of an action potential because the myelin sheath acts as an electrical shield. Therefore, the action potential of normal adult myelinated axons shows little or no sensitivity to 4-aminopyridine at concentrations below 100 µM (9.4 µg/mL) (Shi and Blight, 1997). Concentrations above 1 mM (94.1 µg/mL) tend to cause gradual depolarization of the axon resting potential, perhaps by interacting with leakage channels (Shi and Blight, 1997).

However, when the axon is demyelinated, the internodal membrane and its ion channels become exposed to larger electrical transients during the action potential. Leakage of ionic current through the K+ channel, under these conditions, can contribute to the phenomenon of action potential conduction block (Waxman and Ritchie, 1993). Without being bound by theory, it is understood that 4-Aminopyridine may prolong nerve action potentials by blocking these exposed channels and inhibiting repolarization (Sherratt *et al*., 1980). This is consistent with the ability of the drug to overcome conduction block and increase the safety factor for conduction in some critically demyelinated axons (Bostock *et al*., 1981; Targ and Kocsis, 1985) including those in chronically injured and partially demyelinated mammalian spinal cord (Blight, 1989; Shi and Blight, 1997). An additional study (Shi *et al.,* 1997) showed that this effect of 4-aminopyridine in the chronically injured spinal cord of guinea pigs occurs at a concentration threshold between 0.2 to 1 µM (19.1 to 94.1 ng/mL), though in this tissue it is most effective at about 10 µM (941 ng/mL).

It is understood that blockade of K+ currents amplifies synaptic transmission throughout the brain and spinal cord.

A range of neurological effects occurs with increasing concentrations of 4-aminopyridine in the central nervous system (CNS), up to and including the initiation of seizures. Seizure activity in animals has been seen following large doses of 4-aminopyridine, and seizure activity is part of the toxicological profile of the drug. Synchronous bursting activity in the spinal cord of decerebrate cats has been recorded following administration of very large doses of 4-aminopyridine (5 to 20 mg/kg), which would be expected to produce plasma levels in the region of several hundred ng/mL (Dubuc *et al*., 1986).

Repetitive impulse activity, either spontaneous or in response to single stimuli, occurs in some demyelinated axons exposed to higher levels [0.1 to 1 mM (9.4 to 94.1 µg/mL)] of 4-aminopyridine *in vitro* (Blight, 1989; Bowe *et al.,* 1987; Targ and Kocsis, 1985). A similar effect at lower concentrations (on susceptible neurons or nerve endings) may explain the paresthesias and pain in the area of intravenous infusion reported as side effects of clinical exposure to 4-aminopyridine in human subjects.

Absorption - 4-Aminopyridine is rapidly absorbed following oral administration. In an *in situ* study, 4-aminopyridine was more rapidly absorbed from the small intestine than from the stomach. The absorption half-life was 108.8 minutes and 40.2 minutes for the stomach and small intestine, respectively.

Following oral administration of (non-sustained release) 4-aminopyridine in animals, peak plasma concentrations occur within 1 hour of dosing (FIG. 15). Based on comparisons of the areas under the plasma concentration-versus-time curve (AUC_{(0-∞)}) following i.v. and p.o. administration of 4-aminopyridine (2 mg/kg), the bioavailability of 4-aminopyridine was reported to be approximately 66.5% in male rats and 55% in female rats (M 2001-03). Following oral administration, peak plasma concentrations were 38% lower in females than in males, although both (AUC_{(0-∞)}) and body weight were similar; AUC values did not differ between males and females following i.v. administration.

Studies were performed in rats and dogs using ¹⁴C-labeled 4-aminopyridine (1 mg/kg) given as a single oral gavage dose in solution. In both species, ¹⁴C 4-aminopyridine was rapidly absorbed. Peak plasma levels were achieved within 0.5 to 1 hour in both species. The peak plasma levels (Cmax) and the extent of absorption as reflected by the AUC were both approximately four-fold higher in the dog than in the rat following doses equal on a mg/kg basis. In these studies, there were no gender differences evident in either species. These results are summarized in Table 1.

**Table 1: Summary of Absorption Data for Rats and Dogs Following Single Oral Administration of ¹⁴C-4-Aminopyridine 1 mg/kg (Study Nos. HWI 6379-101 and HWI 6379-102)**

| **Parameter** | **Rats** | | **Dogs** | |
|---|---|---|---|---|
| | **(Study HWI 6379-101)** | | **(Study HWI 6379-102)** | |
| | **Males (N=3¹)** | **Females (N=3¹)** | **Males (N=3)** | **Females (N=3)** |
| Cₘₐₓ (µg/g) | 0.189±0.0202 | 0.168 ± 0.0157 | 0.574 ± 0.1230 | 0.635 ± 0.1028 |
| Tₘₐₓ (hr) | 1.0 | 0.5 | 1.0 ± 0 | 0.8 ± 0.3 |
| AUC (µg·hr/mL) | 0.498 ± 0.0176 | 0.506 ± 0.0633 | 2.03 ± 0.406 | 1.92 ± 0.150 |
| t_{½} (hr) | 1.1 ± 0.04 | 1.4 ± 0.17 | 2.1 ± 0.14 | 1.8 ± 0.04 |

| | | | | |
|---|---|---|---|---|
| 1. Per time point | | | | |

When administered orally, 4-aminopyridine is completely absorbed from the gastrointestinal tract. The absolute bioavailability of two formulations of IR tablets was reported to be 95% (Uges *et al.,* 1982). Absolute bioavailability of Fampridine-SR tablets has not been assessed, but relative bioavailability (as compared to an aqueous oral solution) is 95%. Absorption is rapid unless administered in the context of some feature that achieves delayed release. Such feature may include in a modified matrix containing the 4-AP (*e.g*., Fampridine-SR), or a capsule around any form of 4-AP (sustained or immediate release) that achieves delayed release of the ingredient therein.

When a single Fampridine-SR tablet 10 mg dose is administered to healthy volunteers while in a fasted state, mean peak concentrations ranging in different studies from 17.3 ng/mL to 21.6 ng/mL occurred 3 to 4 hours post-administration (Tₘₐₓ). In comparison, the Cₘₐₓ achieved with the same 10 mg dose of a 4-aminopyridine oral solution was 42.7 ng/mL, which occurred approximately 1.1 hours after dose administration. Exposure increases proportionally with dose (linear kinetics), and steady state maximum concentrations are approximately 29-37% higher than for single doses.

Table 2 illustrates the dose proportionality of 10 mg and 25 mg single doses and the relative bioequivalence of a solid oral dosage form and oral solution.

**Table 2: Relative Bioavailability/Bioequivalence Summary Study Results Conducted in Healthy Adult Volunteers (N=26 with Data)**

| **Parameter** | **Dose** | | | **10 mg *vs*. solution** | | **10 mg *vs*. 25 mg (dose-adjusted)** | |
|---|---|---|---|---|---|---|---|
| | **Fampridine SR Tablet Dose** | | **Buffered Solution (0.83 mg/mL)** | **Ratio of Geometric Means*** | **90% CI** | **Ratio of Geometric Means*** | **90% CI** |
| | **10 mg** | **25 mg** | **10 mg** | | | | |
| Ln-Cₘₐₓ | 2.91 | 3.77 | 3.73 | 43.6 | 41.07-46.35 | 104.3 | 98.07-110.88 |
| Ln-AUC₍₀₋ₜ₎ | 5.21 | 6.09 | 5.35 | 86.7 | 80.60-93.26 | 102.1 | 94.96-109.99 |
| Ln-AUC_{(0-inf)} | 5.37 | 6.17 | 5.42 | 94.7 | 88.23-101.55 | 110.9 | 103.20-119.25 |

The dose proportionality of exposure following single doses of Fampridine-SR is illustrated in Table 3. The pharmacokinetic disposition following of multiple doses of Fampridine-SR is illustrated in Table 4.

**Table 3: Dose-Normalized Pharmacokinetic Parameter Values (Mean ± SEM) Following Single Oral Administration of Fampridine-SR Tablets to Patients with MS**

| **Parameter** | **Single Dose (mg)** | | | |
|---|---|---|---|---|
| | **5** | **10** | **15** | **20** |
| | **(n=24)** | **(n=24)** | **(n=24)** | **(n=23)** |
| Cₘₐₓ-norm* (ng/mL) | 13.1 ± 0.6 | 12.6 ± 0.7 | 12.3 ± 0.7 | 12.3 ± 0.8 |
| Tₘₐₓ (hours) | 3.9 ± 0.2 | 3.9 ± 0.3 | 3.6 ± 0.3 | 3.6 ± 0.3 |
| AUC-norm* (ng·hr/mL) | 122.1 ± 9.4 | 122.1 ± 9.4 | 131.5 ± 7.4 | 127.8 ± 6.9 |
| t_{½} (hours) | 5.8 ± 0.5 | 5.6 ± 0.4 | 5.5 ± 0.4 | 5.1 ± 0.3 |
| Cl/F (mL/min) | 619.8 ± 36.2 | 641.4 ± 39.1 | 632.4 ± 39.0 | 653.9 ± 37.1 |

| | | | | |
|---|---|---|---|---|
| * Normalized to a 5 mg dose. | | | | |

**Table 4: Pharmacokinetic Parameter Values (Mean and 95% CI) Following Multiple Oral Doses of Fampridine-SR Tablets (40 mg/day, 20 mg b.i.d.) in 20 Patients with MS**

| **Day** | **Parameter - Multiple Doses** | | | | |
|---|---|---|---|---|---|
| | **Cₘₐₓ (ng/mL)** | **Tₘₐₓ (hours)** | **AUC₍₀₋₁₂₎ (ng·hr/mL)** | **t_{½} (hours)** | **Cl/F (mL/min)** |
| Day 1 | 48.6 (42.0, 55.3) | 3.8 (3.2, 4.3) | NE | NE | NE |
| Day 7/8 | 66.7 (57.5, 76.0) | 3.3 (2.8, 3.9) | 531 (452,610) | NE | 700 (557, 844) |
| Day 14/15 | 62.6 (55.7, 69.4) | 3.3 (2.6, 3.9) | 499 (446, 552) | 5.8 (5.0, 6.6) | 703 (621, 786) |

| | | | | | |
|---|---|---|---|---|---|
| NE = Not evaluable | | | | | |

Distribution - The volume of distribution at steady state (V_{dss}) in rats has been reported to approximate total body volume (not adjusted for bioavailability). Following administration of a single p.o. dose of 4-aminopyridine (2 mg/kg) to male and female rats, V_{dss} is 13% lower in females than in males (1094.4 mL in males versus 947.5 mL in females); however, the difference is not statistically significant. Furthermore, when adjusted for body weight differences, there is no difference between males and females (2%).

In a single-dose study, rats were administered ¹⁴C-labeled 4-aminopyridine (1 mg/kg) p.o. Three animals per time point were sacrificed 1, 3, 8, and 24 hours post-dose. Blood was collected and tissues were excised for determination of radioactivity. One hour post-dose, at a time approximately corresponding to the peak plasma concentration, radioactivity was detected in all tissues collected. The amounts represented small percentages of the dose; however, only 58.3% of the dose was accounted for in total. The highest concentrations were in the liver (2.6%), kidney (1.6%), and blood (0.7%); 51 % of the radioactivity was in the carcass (primarily the gastrointestinal tract and musculoskeletal system). The half-life of elimination from tissues ranged from 1.1 to 2.0 hours. By 3 hours post-dose, the amount of radioactivity detected in all tissues was negligible (with the exception of the carcass, which contained 15.4% of the radioactive dose).

An *in vitro* study was conducted to assess plasma protein binding in rat and dog plasma. 4-Aminopyridine concentrations of 5, 50, or 500 ng/mL were used. 4-Aminopyridine was largely unbound and had a high free drug fraction at all three concentrations tested. After a 4-hour dialysis period, the mean percent of free drug ranged from 73 to 94% in rat plasma and 88 to 97% in dog plasma.

4-aminopyridine is largely unbound to plasma proteins (97 to 99%). Administration of a single 20 mg intravenous dose, mean Vd is 2.6 L/kg, greatly exceeding total body water (Uges *et al.,* 1982), this is similar to values calculated in healthy volunteers and in patients with SCI who received Fampridine-SR tablets. The plasma concentration-time profile is one of two or three compartments with a rapid initial distribution phase. Measurable levels are present in the saliva.

In the rat, ¹⁴C-labeled 4-aminopyridine was detected in the cerebrum and cerebellum at tissue-to-blood ratios of 3.07 and 1.48, respectively, indicating that 4-aminopyridine crosses the blood brain barrier following an oral dose. 4-aminopyridine is eliminated from the brain at a similar rate as from the blood. Specifically, the elimination half-lives of 4-aminopyridine from brain tissues (cerebellum and cerebrum) and the blood are similar (1.24, 1.63, and 1.21 hours, respectively).

Toxicology - In single- and repeated-dose toxicity studies, the dosing regimen greatly affected the rate of side effects. Clinical signs evident after large single doses or repeated lower doses were similar in all species studied and included tremors, convulsions, ataxia, dyspnea, dilated pupils, prostration, abnormal vocalization, increased respiration, excess salivation, gait abnormalities, and hyper- and hypo-excitability. These clinical signs were not unexpected and represent exaggerated pharmacology of 4-aminopyridine.

In controlled clinical studies involving the use of 4-aminopyridine, the most frequent adverse events by body system occurred in the nervous system, "body as a whole", and digestive system. Dizziness, insomnia, paresthesia, pain, headache and asthenia are the most common nervous system adverse events, and nausea is the most frequently reported event in the digestive system category.

The most frequent treatment-related adverse events that have been reported with Fampridine-SR, in MS patients as well as other populations including spinal cord injury, may be broadly categorized as excitatory effects in the nervous system, consistent with the potassium channel blocking activity of the compound. These adverse events include dizziness, paresthesias, insomnia, balance disorders, anxiety, confusion and seizure. While an increased incidence of such events appears to be moderately dose-related, the susceptibility of individuals is quite variable. As a result of disease pathology, there appears to be potential for more lowering of seizure threshold in people with MS than for people with spinal cord injury; this may result from interaction of the channel-blocking properties of the drug with MS brain pathology in certain individuals.

Formulations and Administration - It is especially advantageous to formulate parenteral compositions in dosage unit form for ease of administration and uniformity of dosage. Dosage unit form as used herein refers to physically discrete units suited for administration to the subjects to be treated; each unit containing a predetermined quantity of therapeutic compound calculated to produce the desired therapeutic effect in association with any pharmaceutical carrier. Administration can be of a single dosage unit form, or administration can be of multiples of dosage unit forms, including concurrent administration of unit doses of various amounts.

In general, dosage unit forms of the invention are dictated by and directly dependent on the unique characteristics of the therapeutic compound and the particular therapeutic effect to be achieved in a given patient or patient population. Unit dosage forms can be tablets, capsule, aliquots; unit does forms can be provided as blister packs containing one or more dose. In certain administration protocols a patient may utilize more than a single unit dose at a time, *e.g*., consume two capsules, or two tablets contained in separate blisters of a blister pack.

Active compounds are administered at a therapeutically effective dosage sufficient to treat a condition associated with a particular state in a patient. A "therapeutically effective amount" preferably reduces or ameliorates symptoms of the condition, or the condition itself, in the patient by at least about 20%, more preferably by at least about 40%, even more preferably by at least about 60%, and still more preferably by at least about 80% relative to untreated subjects. The efficacy of a compound can be based on evaluation in an animal model system that may be predictive of efficacy in treating the disease in humans, such as the model systems described herein. The efficacy of a compound can be based on results found in a normative population, such as from a clinical trial.

The actual dosage amount of the 4-AP administered to a subject may be determined by physical and physiological factors such as age, sex, body weight, severity of condition, the type of disease being treated, previous or concurrent therapeutic interventions, idiopathy of the subject and on the route of administration. Alternatively, a standard amount can be provided to essentially all patients, often the standard is dervied from studies in normative population such as from data on human subjects in clinical trials. These factors may be determined by a skilled artisan, such as a health care provider, medicine prescriber, physician, pharmacist, etc. (collectively "practitioner"). In certain embodiments, the practitioner responsible for administration may determine the concentration of active ingredient(s) in a composition and appropriate dose(s) for an individual subject. In certain embodiments, the dosage may be adjusted by the individual physician in the event of any complication. ; in certain embodiments, a dose is administered to all patients (regardless of MS classification, temperature sensitivity, duration of disease, progressive status, etc.) at an amount that is found to be safe and effective in a normative reference population.

Sustained release formulations and compositions of the present invention exhibit a desired release profile that may be described in terms of the maximum plasma concentration of the drug or active agent at steady state (C_{maxSS}) and the minimum plasma concentration of the drug or active agent at steady state (C_{minSS}). Steady state is observed when the rate of administration (absorption) is equal to the rate of elimination of the drug or active agent. A ratio of C_{maxSS} to CminSS (C_{maxSS}:C_{minSS}) may be calculated from the observed C_{maxSS} and C_{minSS}. In addition, the formulations and compositions of the present invention exhibit a desired release profile that may be described in terms of the average maximum plasma concentration of the drug or active agent at steady state (C_{avSS}).

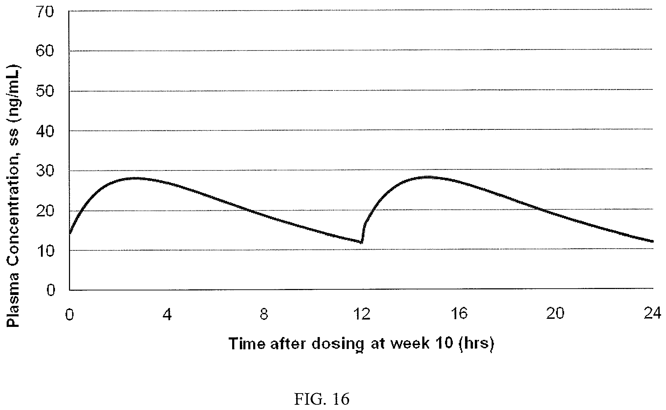

As set forth herein, sustained release 4-aminopyridine composition exhibits a C_{maxSS}:C_{minSS} ratio from about 1.0 to 3.5 for either twice daily (BID) or once daily (QD) administration. In alternative embodiments, a sustained release formulation comprises a C_{maxSS}:C_{minSS} ratio of about 1.5 to about 3.0 for either twice daily (BID) or once daily (QD) administration. In another preferred embodiment, the C_{maxSS}:C_{minSS} ratio is about 2.0 to about 3.0 for either twice daily (BID) or once daily (QD) administration. As is readily understood by those of ordinary skill in the art, when redosing occurs at a time when plasma levels are decreasing, C_{minSS} occurs at the time of redosing. The foregoing can be exemplified by referring to Figure 16, which is a steady state pharmacokinetic profile for sustained release 4AP.

A further aspect is a sustained release composition comprising a sustained release matrix and an aminopyridine, wherein said composition provides a CavSS of about 15 ng/ml to about 35 ng/ml. In a further aspect, a sustained release tablet comprising a sustained release matrix and an aminopyridine, said tablet exhibiting a CmaxSS of about 20 ng/ml to about 35 ng/ml is provided. The pharmacokinetic characteristics of sustained release aminopyridine compositions and methods of treating various neurological disorders are described in co-pending U.S. application Ser. No. 11/102,559 entitled "Stable Formulations of Aminopyrdines and Uses Thereof" filed Apr. 17, 2004 and U.S. application Ser. No. 11/010,828 entitled "Sustained Release Aminopyridine Composition" filed Dec. 13, 2004, the contents of which are incorporated herein by reference in their entireties.

Kits - Kits comprise an exemplary embodiment of the invention. The kit can comprise an outer receptacle or container configured to receive one or more inner receptacles/containers, utensils and/or instructions. A utensil in accordance with the invention can comprise item(s) to administer the drug, such as a patch, inhalation apparatus, fluid container cup, syringe or needle. A composition of the invention can be comprised within some receptacle or container of the invention. A receptacle of the invention can contain sufficient quantity of a composition of the invention to be useful for multiple doses, or may be in unit or single dose form.

Kits of the invention generally comprise instructions for administration in accordance with the present invention. Any mode of administration supported herein can constitute some portion of the instructions. In one embodiment, the instructions indicate that the composition of the invention is to be taken twice daily. The instructions may be affixed to any container/receptacle of the invention. Alternatively, the instructions can be printed on or embossed in/on or formed as a component of a receptacle of the invention.

A kit may also include instructions for employing the kit components as well the use of any other reagent not included in the kit. It is contemplated that such reagents are embodiments of kits of the invention. Such kits, however, are not limited to the particular items identified above and may include any reagent used directly or indirectly in the treatment of cognitive dysfunction or cognitive impairment.

In one embodiment a kit of the invention includes, e.g., a bottle, with drug of the invention therein, accompanied by information for dosing and/or safety; the information may be affixed to the bottle or provided concurrently such that the patient obtains the bottle and instructions essentially concurrently. In one embodiment a kit of the invention includes, e.g., a blister pack with drug of the invention therein, accompanied by information for dosing and/or safety; the information may be affixed to the bottle or provided concurrently such that the patient obtains the bottle and instructions essentially concurrently. Generally, drug of the invention in a kit is in unit dose form.

Durable Treatment - Embodiments of the present invention comprise methods of effectively treating multiple sclerosis in a patient over a chronic or extended or prolonged or protracted or sustained time period; this is also referred to as a "durable" treatment or a "durable" method of treatment; this is also referred to as a "sustained" treatment or a "sustained" method of treatment. Another embodiment of the present invention is directed to methods of maintaining improvement of a symptom of multiple sclerosis in a patient comprising administering a therapeutically effective amount of 4-aminopyridine to said patient after previously achieving an improvement of a symptom of multiple sclerosis in said patient during contiguous or continuing or prior administration of 4-aminopyridine. Any of such methods comprise administering a therapeutically effective amount of 4-aminopyridine to said patient for an extended, prolonged, protracted, sustained or chronic period of time (as used herein, extended, prolonged, protracted, sustained, chronic are synonyms unless the context clearly indicates otherwise).

In certain embodiments, the extended, prolonged, protracted, sustained or chronic period is at least or more than: 8,9, 10, 11, 12, 13, 14, 15, 16, 17, 18, 19, 20, 21 or 22 weeks; 3, 4,5, 6, 7, 8, 9, 10, 11, 12, 13, 14, 15, 16, 17, or 18 months; or 1, 2, 3, 4, 5, 6, or greater than 5 years. In certain embodiments, the extended, prolonged, protracted, chronic or sustained period is for the lifetime of the patient. These methods can also comprise administering the 4-aminopyridine at or to a therapeutic level (such as Cminss or an average Cminss) or range (such as a Cminss range or a reference range of average Cminss values) in accordance with the present invention.

In certain embodiments, the improved symptom is not walking, not walking ability, not increased or improved walking speed, not cognition and/or not spasticity. That is in certain embodiments one or more of these parameters may or may not be specifically excluded.

In certain embodiments, the therapeutically effective amount of 4-aminopyridine is 10 milligrams in a sustained release composition administered twice daily. In certain embodiments, the sustained release composition may be administered twice daily. In certain embodiments, the sustained release composition may be administered once daily. These methods can also comprise administering the 4-aminopyridine at or to a therapeutic level (such as Cminss) or range (such as a Cminss range) in accordance with the present invention. Certain embodiments are directed to the use of 4-aminopyridine for treatment of a demyelinating condition for at least 8, 9, 10, 11, 12, 13, 14, 15, 16, 17, 18, 19, 20, 21 or 22 weeks; 3, 4,5, 6, 7, 8, 9, 10, 11, 12, 13, 14, 15, 16, 17, or 18 months; or 1, 2, 3, 4, 5, 6, or greater than 5 years. Still further embodiments are directed to the use of 4-aminopyridine in preparing a medicament or therapeutic or formulation for chronic or durable treatment of a demyelinating condition or multiple sclerosis or traumatic neuronal injury.

Further embodiments of the present invention are directed to methods of achieving sustained or relatively sustained improvement in a symptom of MS in a patient with multiple sclerosis, comprising continuing administration of a therapeutically effective amount of 4-aminopyridine to said patient over an extended period of time. Improvement is generally defined with regard to a control or standard amount or value; it is understood that there is often progressive decline in patients with a disease such as multiple sclerosis so that an increase or relative increase can properly be considered in regard to the decline in function attendant to the inherent progress of multiple sclerosis pathology.

In certain embodiments, the therapeutically effective amount of 4-aminopyridine is 10 milligrams in a sustained release composition. In certain embodiments, the sustained release composition can be administered twice daily. In certain embodiments, the sustained release composition may be administered once daily. These methods can also comprise administering the 4-aminopyridine at or to a therapeutic level (such as not going below Cminss) or range (such as staying within a Cminss - Cmaxss range) in accordance with the present invention.

Methods of the invention also comprise achieving sustained improvement in a symptom of MS in a patient comprising continuing administration a therapeutically effective amount of 4-aminopyridine to said patient over an extended period of time. This sustained improvement can be relatively growing in that there is an ongoing growth in a percentage improvement elative to a reference or normative population, or this improvement can be relatively varied in that there is a fluctuating percentage improvement elative to a reference or normative population such that there is a tendency to do better than the reference group; when the improvement is relatively varied this can include periods when the subject patient may do worse relative to a reference or normative population.

Combination treatments - The compositions and methods of the present invention may be used in the context of a number of therapeutic or prophylactic applications. In order to increase the effectiveness of a treatment with the compositions of the present invention, e.g., aminopyridines, or to augment the protection of another therapy (second therapy), it may be desirable to combine these compositions and methods with other agents and methods effective in the treatment, amelioration, or prevention of diseases and pathologic conditions, for example, dysfunctions or impairments produced by demyelination of nerve cells.

Administration of a composition of the present invention to a subject will follow general protocols for the administration described herein, and the general protocols for the administration of a particular secondary therapy will also be followed, taking into account the toxicity, if any, of the treatment. It is expected that the treatment cycles would be repeated as necessary. It also is contemplated that various standard therapies may be applied in combination with the described therapies.

Various combinations may be employed; for example, an aminopyridine or derivative or analog thereof, is "A" and the secondary therapy (e.g., an immune system modulator often used in MS) is "B", nonlimiting combination cycles include (these are merely exemplary and the order may be defined in a forward or backward direction) :

| | | | | | | | |
|---|---|---|---|---|---|---|---|
| | A/B/A | B/A/B | B/B/A | A/A/B | A/B/B | B/A/A | A/B/B/B |
| B/A/B/B | | | | | | | |
| | B/B/B/A | B/B/A/B | A/A/B/B | A/B/A/B | | A/B/B/A | B/B/A/A |
| | B/A/B/A | B/A/A/B | A/A/A/B | B/A/A/A | | A/B/A/A | A/A/B/A |
| | AB/AB | A/AB | AB/B | AB | | A/B | |
| | AB/AB/AB | AB/B/B | AB/B/AB | AB/A/A | | | |

### EXAMPLES

### Example 1: Long-term Use of 4-AP, The MS-F203 Extension Study (MS-F203EXT)

An interim assessment of efficacy and safety of oral sustained release fampridine (Fampridine-SR) in patients with multiple sclerosis (MS) participating in an ongoing open label extension study has been obtained.

Background: A Phase 3 study (MS-F203) of fampridine in MS showed improvement in walking ability. This extension study (MS-F203EXT) monitored efficacy and safety during open-label treatment of patients from MS-F203. Design/methods: Patients were treated with 10 mg fampridine bid, and were assessed in the clinic at 2, 14, and 26 weeks and every 6 months thereafter. Eligibility criteria included: definite MS; age 18-70; Timed 25-Foot Walk (T25FW) times of 8-45 seconds at screening for MS-F203. Efficacy was analyzed by the proportion of Extension Timed Walk Responders (ETWRs) whose walking was faster during the majority of visits than their fastest off-treatment speed.

Interim data from a long-term extension study (MS-F203-EXT) of a Phase 3 Fampridine-SR trial (MS-F203), showed that 24.9% of extension study participants met the criteria as Extension Timed Walk Responders (ETWR) after one year of treatment and furthermore demonstrated improved walking speed over at least a two-year period. In addition, the safety profile of Fampridine-SR observed over two years in this study was consistent with previous placebo-controlled trials.

Prior to the extension study, the Phase 3 study (MS-F203) of 4-aminopyridine in MS showed improvement in walking ability; MS-F203 was a Double-Blind, Phase 3 Study, In MS-F203 patients were randomized in 3:1 ratio to 10 mg bid Fampridine-SR or placebo. The primary outcome was consistent improvement in walking speed on the Timed 25 Foot Walk (Timed Walk Response).

Design/methods: MS-F203EXT monitored efficacy and safety during open-label treatment of patients from MS-F203. Patients were treated with 10 mg fampridine bid, and were assessed in the clinic at 2, 14, and 26 weeks and every 6 months thereafter. Eligibility criteria included: definite MS; age 18-70; Timed 25-Foot Walk (T25FW) times of 8-45 seconds at screening for MS-F203. Efficacy was analyzed by the proportion of Extension Timed Walk Responders (ETWRs) whose walking was faster during the majority of visits than their fastest off-treatment speed. The MS-F203 EXT study is ongoing as of this filing. There are safety assessments, a timed 25 Foot Walk assessment, Clinician and Subject Global Impression at each visit. An EDSS assessment is performed every 2 years. The timeline and exposure (with an interim data cutoff of Nov 30, 2008) of the MS-F203 EXT study is as follows: The first patient enrolled 13 December 2005. The average exposure is 2.1 years (range 9 days - 3 years). The total exposure is 565 patient-years.

In the 14-week placebo-controlled MS-F203 study, 34.8% of subjects met the outcome criteria defined as Timed Walk Responders in the Fampridine-SR group compared to 8.3% of subjects in the placebo group. Following the placebo-controlled study, 269 of the 283 participants who completed the study (the 269 included those defined as Timed Walk Responders, non-responders and participants from the placebo group) enrolled in the open label extension study (MS-F203EXT). All participants in the extension study were treated with Fampridine-SR at 10 mg twice daily, and assessed in the clinic at 2, 14, 26, 52, 78 and 104 weeks.

Of the 269 patients completing MS-F203, enrolled in MS-F203EXT, 263 had at least one on-treatment evaluation. At this interim analysis, patients had been treated up to 2.6 years (average exposure of 1.9 years) 196 remained active, 24 discontinued for adverse events and 59 experienced at least one serious adverse event, the most common being MS relapse. A total of 66 (24.9%) patients were ETWRs and showed a mean improvement in walking speed of >30% at each visit over the first year of treatment. By 24 months, the mean improvement decreased to 22%. By contrast, Extension Timed Walk Non-responders showed a decline in mean T25FW speed of 8% over two years. There was a statistically significant improvement in Subject and Clinician Global Impression scores for ETWRs compared to Non-responders (p < 0.005).

Timed walk response in the extension study was measured using the Timed 25-Foot Walk (T25W). An Extension Timed Walk Responder (ETWR) was defined as one walking faster in the majority of the first four open-label visits (2, 14, 26 and 52 weeks) compared to the fastest off-treatment speed, taken from amongst measurements at five separate time points during the placebo-controlled trial and again at the beginning of the extension study.

At the time of an interim analysis, participants had been treated for up to 3 years, with an average exposure of 2.1 years and a total exposure of 565 patient-years. A total of 187 of the 269 (69.7%) subjects who enrolled in the extension trial were still enrolled at the time of the interim analysis. More than half of the study participants were diagnosed with secondary-progressive MS (52.8%), with the remainder of diagnosed with relapsing-remitting MS (28.6%), primary-progressive MS (14.9%) and progressive relapsing (3.7%).

Results - A total of 66 of 269 (24.9%) of study participants were Extension Timed Walk Responders (ETWRs) after one year of treatment with Fampridine-SR 10 mg twice daily. ETWRs showed a mean improvement of >30% in walking speed visit after 12 months of treatment and 22% improvement at 24 months compared to their best off-treatment speed. Extension Timed Walk Non-Responders showed a decline in mean walking speed of 8% over 24 months. There were also statistically significant improvements in patient and clinician global impression scales for ETWRs compared to Non-Responders (p<0.005).

Among participants defined as Timed Walk Responders in the placebo-controlled trial, 42.9% met the criteria for ETWRs after one year in the extension study. In addition, 16.2% of subjects receiving placebo in the placebo-controlled MS-F203 trial met the criteria as ETWRs in the extension and 16.2% of subjects receiving placebo in the placebo-controlled trial met the criteria for ETWRs. Notably, 19.7% of participants defined as Timed Walk Non-Responders in the placebo-controlled MS-F203 trial later met the criteria as ETWRs in the extension.

All study participants were evaluated for overall disability using the Expanded Disability Status Score (EDSS) at the beginning of the placebo-controlled trial, and then reassessed after two years in the extension study. The mean EDSS score at baseline as 5.76, representing significant disability. After two years in the extension study, the change from EDSS baseline in ETWRs was -0.1 compared to +0.4 in non-responders (p=0.018). Increases in EDSS scores indicate a worsening of disability.

Conclusions - The retention Rate in the Open-Label Study was 82.9% after one year and 75% after two years. In addition, sustained walking speed improvement was seen after two years in Responder Group. MS patients treated with 4-aminopyridine showed consistently improved and *clinically meaningful* walking speed during 2 years of open-label treatment. This study provides the first evidence of such long term efficacy of treatment with 4-aminopyridine. No new safety issues emerged.

Thus, there has been a high retention rate in open-label studies: 82.9% at 1 year, 75.0% at 2 years. A subset of patients experienced sustained improvement in walking speed over 2 years. There have been no new safety signals. The incidence of seizure at 10 mg bid was consistent with expected background rates and other MS trial experience.

Fampridine-SR is seen to be safe and useful for long term use. In particular the drug was found to be useful, *e.g*., for consistent improvement in walking speed on the Timed 25 Foot Walk (Timed Walk Response).

These long-term data for Fampridine-SR are important because this medicine is used as a chronic therapy for people with MS. The data indicate that Fampridine-SR produced a sustained, clinically meaningful improvement in walking speed for a subset of people with MS over a two-year period.

A subset of MS patients treated with fampridine showed *consistently improved walking speed*, this improvement was deemed clinically meaningful, during 2 years of open-label treatment. This is the first disclosure in the art of data establishing long-term efficacy and safe and clinically meaningful treatment with fampridine. No new safety issues emerged.

### Further detail: MSF-203EXT

This is a long-term, multi-center, open-label extension study of continued treatment with Fampridine-SR. The target population consisted of patients throughout the United States and Canada who had been diagnosed with multiple sclerosis and who had participated in study MS-F203.

A total of 269 patients were enrolled and are included in the present study. Patients were screened for the present study at the Final Visit (Visit 8) of the MS-F203 study, and, upon meeting the inclusion and exclusion criteria, were dispensed open-label investigational drug for MS-F203 EXT at that visit. If a patient was not screened for this study at the same time as completing the final visit for the MS-F203 study, a separate full Screening Visit was required at study entry. Patients who qualified at that Screening Visit were asked to return within two weeks for an additional visit (Visit 0) in order to receive their first open-label investigational drug. The schedule of visits thereafter was the same for all patients. Patients returned 2 weeks later for Visit 1 and 12 weeks after that for Visit 2. Visit 3 was to occur 12 weeks after Visit 2. Thereafter, patients were to be seen every 26 weeks, with two telephone visits approximately 8 weeks and 16 weeks after each clinic visit. At the end of the study, patients returned for their next regularly scheduled 26-week visit (the Final Visit), and began a four-week follow-up period in which they take no investigational drug. They are to return in 4 weeks for follow-up assessments (the Follow-up Visit).

Patients took one tablet every 12 hours throughout the study at approximately the same time each day. All patients received a stable treatment dose of 10 mg BID (20 mg/day) of Fampridine-SR. Functional assessments included the
- Timed 25 Foot Walk,
- Clinician Global Impression of Change (CGI),
- Subject Global Impression (SGI), and
- Expanded Disability Status Score (EDSS).

Safety evaluations were based on adverse event reporting, physical examination, clinical laboratory testing, 12-lead ECG and standard EEG testing (required of all patients at the Screening Visit; should be obtained if a patient has a seizure.) At each clinic visit, the Investigator was required to document whether or not continued participation in the study was in the patient's best interest. Patients terminating therapy were to undergo an Early Termination Visit.

Diagnosis and main criteria for inclusion - For inclusion into the trial, patients were required to have
- participated in study MS-F203 (receiving either Fampridine-SR or placebo),
- to have had clinically definite multiple sclerosis as defined by McDonald criteria, and,
- male or female, must have been at least 18 years of age.

Any patient who was over the age of 70 was in good overall health in the judgment of the Investigator.

Test product, dose and mode of administration - The test product was Fampridine SR 10 mg tablets administered orally b.i.d. (every 12 hours).

Duration of treatment and estimated patient-year exposure - This is an ongoing study. Based on ongoing monitoring, the 269 patients in the study have been exposed to Fampridine-SR 10 mg b.i.d. for approximately 2.1 years on average (range = 8.5 days, 3.0 years). The estimated exposure with the clinical cutoff of 30 Nov 2008 for this cohort is approximately 565 patient-years.

Efficacy - The functional and subjective efficacy assessments included the Timed 25 Foot Walk, the Clinician Global Impression of Change (CGI), and the Subject Global Impression (SGI).

Safety - Safety evaluations included adverse event reporting, physical examination, clinical laboratory testing, vital signs, and 12-lead ECG.

Statistical methods - This is an abbreviated interim analysis to support the clinical safety update for the New Drug Application of Fampridine-SR in patients with multiple sclerosis. All computations were performed using SAS® Version 8.2 or above. All study data came from the updated pooled database using the 30 November clinical cut-off. Descriptive statistics (n, mean, standard deviation [SD], median, minimum and maximum) were calculated for continuous variables and ordinal variables with more than four categories. Frequencies and percentages were tabulated for all other categorical variables. Percentages were based on the total number of non-missing values. The safety population included all patients who received open-label investigational drug. This population was used in analyses of demographics and baseline characteristics data as well as in the analysis of the safety data. Only one analysis dose group was defined in this study (10 mg b.i.d.). No formal hypothesis testing was performed. Abbreviated safety analyses were conducted with respect to the following variables: treatment-emergent adverse events (TEAEs) and clinically significant: vital signs, laboratory assessments, and ECG parameters

Patient Disposition - Enrolled: N = 269; Number discontinued = 82 (30.5%) (28 due to adverse event and 54 due to other reasons); Number active=187 (69.7%).

Efficacy Results - The updated efficacy results from the ongoing open-label studies are provided in a combined Meta-analysis clinical study report (MS-F-EXT) which includes MS-F202EXT, MS-F203EXT, and MS-F204EXT. This Meta-analysis report examines signals of long-term efficacy of Fampridine-SR in multiple sclerosis patients.

Safety Results - The most common adverse events (TEAE) seen in Fampridine-SR-treated patients in the double-blind, placebo-controlled parent study (MS-F203) were falls, urinary tract infection, dizziness, insomnia, fatigue, nausea, upper respiratory tract infection, asthenia, back pain, balance disorder, and headache. In this extension study (MS-F203EXT), the most common adverse events were urinary tract infection, multiple sclerosis relapse, falls, arthralgia, asthenia, pain in extremities, nausea, peripheral edema, upper respiratory tract infection, and insomnia. In general, the adverse event pattern observed in this open-label extension study sub-population is similar to the adverse event pattern recorded for Fampridine-SR treated patients in the double-blind studies. Among the 269 patients who were treated, 187 remained ongoing as of 30 Nov 2008. Twenty-eight patients were withdrawn from the study due to adverse events. Additionally, four patients were withdrawn for non-compliance, 27 withdrew consent, four were lost to follow-up, and 19 were withdrawn for 'Other' reasons. Sixty-three patients experienced a serious adverse event at some point in the study, the most common being multiple sclerosis relapse. In addition to adverse events summary tables, patient accounting, demographics, and clinically significant: laboratory values, vital signs, and ECG summary tables are presented at the end-of text. One patient experienced a complex partial seizure and three patients experienced generalized seizure (convulsion) while on 10 mg b.i.d. Fampridine-SR.

This ongoing extension study provided additional evidence of the long term efficacy and tolerability of Fampridine-SR in patients with multiple sclerosis. The retention of 70% of patients after nearly 3 years of study attests to the perceived benefit and safety of the treatment on the part of patients and investigating physicians.

### Example 2: Long-term Use of 4-AP - The MS-F204 Extension Study (MS-F204EXT)

This example provides data for is a long-term, multi-center, open-label extension study of continued treatment with Fampridine-SR. The target population consisted of patients throughout the United States and Canada who had been diagnosed with multiple sclerosis and who had participated in study MS-F204.

A total of 214 patients were enrolled and are included in the present study. Patients were screened at the Final Visit (Visit 8) of the MS-F204 study, and, upon meeting the inclusion and exclusion criteria, were dispensed open-label investigational drug for MS-F204 EXT at that visit. If a patient was not screened at the same time as completing the final visit for the MS-F204 study, a separate full Screening Visit was required at study entry. Patients who qualified at that Screening Visit were asked to return within two weeks for an additional visit (Visit 0) in order to receive their first open-label investigational drug. The schedule of visits thereafter was the same for all patients. Patients returned 2 weeks later for Visit 1 and 12 weeks after that for Visit 2. Visit 3 occurred 12 weeks after Visit 2. Thereafter, patients were seen every 26 weeks, with two telephone visits approximately 8 weeks and 16 weeks after each clinic visit. At the end of the study, patients were returned for their next regularly scheduled 26-week visit (the Final Visit), and began a four-week follow-up period in which they take no investigational drug. They returned in 4 weeks for follow-up assessments (the Follow-up Visit).

Patients took one tablet every 12 hours throughout the study at approximately the same time each day. All patients received a stable treatment dose of 10 mg b.i.d. (20 mg/day) of Fampridine-SR.
- Functional assessments included the
- Timed 25 Foot Walk,
- Clinician Global Impression of Change (CGI),
- Subject Global Impression (SGI), and
- Expanded Disability Status Score (EDSS).

Safety evaluations were based on adverse event reporting, physical examination, clinical laboratory testing, 12-lead ECG and standard EEG testing (required of all patients at the Screening Visit; should be obtained if a patient has a seizure.) At each clinic visit, the Investigator was required to document whether or not continued participation in the study was in the patient's best interest. Patients terminating therapy underwent an Early Termination Visit.

Diagnosis and main criteria for inclusion - For inclusion into the trial, patients were required to have
- participated in study MS-F204 (receiving either Fampridine-SR or placebo),
- to have had clinically definite multiple sclerosis as defined by McDonald criteria, and,
- male or female, must have been at least 18 years of age.

Any patient who was over the age of 70 was in good overall health in the judgment of the Investigator.

Test product, dose and mode of administration - The test product was Fampridine-SR 10 mg tablets administered orally b.i.d. (every 12 hours).

Duration of treatment and estimated patient-year exposure - The 214 patients in the study were exposed to Fampridine-SR 10 mg b.i.d. for approximately 0.9 years on average (range = 7.5 days, 1.3 years). The estimated exposure with the clinical cutoff of 30 Nov 2008 for this cohort is approximately 193 patient-years.

Efficacy - The functional and subjective efficacy assessments included the Timed 25 Foot Walk, the Clinician Global Impression of Change (CGI), and the Subject Global Impression (SGI).

Safety - Safety evaluations included adverse event reporting, physical examination, clinical laboratory testing, vital signs, and 12-lead ECG.

Statistical methods - All computations were performed using SAS® Version 8.2 or above. All study data came from the updated pooled database using the 30 November clinical cut-off. Descriptive statistics (n, mean, standard deviation [SD], median, minimum and maximum) were calculated for continuous variables and ordinal variables with more than four categories. Frequencies and percentages were tabulated for all other categorical variables. Percentages were based on the total number of non-missing values. The safety population included all patients who received open-label investigational drug. This population was used in analyses of demographics and baseline characteristics data as well as in the analysis of the safety data. Only one analysis dose group was defined in this study (10 mg b.i.d.). No formal hypothesis testing was performed. Abbreviated safety analyses were conducted with respect to the following variables: treatment-emergent adverse events (TEAEs) and clinically significant:
- vital signs,
- laboratory assessments, and
- ECG parameters

Patient Disposition - Enrolled: N = 214; Number discontinued = 30 (14.0%) (4 due to adverse event and 26 due to other reasons); Number active = 184 (86.0%).

Safety Results - The most common adverse events (TEAE) seen in Fampridine-SR-treated patients in the double-blind, placebo-controlled parent study (MS-F204) were urinary tract infections, falls, insomnia, headache, asthenia, dizziness, nausea, back pain, balance disorder, upper respiratory tract infection, arthralgia, nasopharyngitis, and paraesthesia. In this extension study (MS-F204EXT), the most common adverse events were falls, urinary tract infections, multiple sclerosis relapse, asthenia, balance disorder, arthralgia, upper respiratory tract infection, pain in extremities, nausea, contusion, dizziness, fatigue, and peripheral edema. In general, the adverse event pattern observed in this open-label extension study sub-population is similar to the adverse event pattern recorded for Fampridine-SR treated patients in the double-blind studies. Among the 214 patients, who were treated, 184 remained ongoing as of 30 Nov 2008. Four patients were withdrawn from the study due to adverse events. Additionally, 15 withdrew consent and one was lost to follow-up and 10 were withdrawn for 'Other' reasons. Seventeen patients experienced a serious adverse event at some point in the study, the most common being multiple sclerosis relapse. In addition to adverse events summary tables, patient accounting, demographics, and clinically significant: laboratory values, vital signs, and ECG summary tables are presented at the end-of text. There has been no report of seizures in the study as of 30 Nov 2008.

Efficacy Results - The updated efficacy results from the ongoing open-label studies are provided in a combined Meta-analysis clinical study report (MS-F-EXT) which includes MS-F202EXT, MS-F203EXT, and MS-F204EXT. This Meta-analysis report examines signals of long-term efficacy of Fampridine-SR in multiple sclerosis patients.

This ongoing extension study has provided additional evidence of the long term efficacy and tolerability of Fampridine-SR in patients with multiple sclerosis. The retention of 86% of patients after more than 15 months of study attests to the benefit and safety of the treatment as seen by both patients and treating physicians.

### Example 3: Interim Analysis of Open-Label Extension Studies (MS-F203EXT and MS-F204EXT) of Fampridine-SR Tablets in Patients with Multiple Sclerosis

This data represents an interim assessment of efficacy and safety of Fampridine-SR, in patients with multiple sclerosis (MS) participating in ongoing, open-label extension studies.

Two Phase 3, double-blind studies (MS-F203 and MS-F204) of Fampridine-SR in MS patients demonstrated improvement in walking speed (WS) using the Timed 25-Foot Walk. Each of these placebo-controlled studies were followed in the open-label extension studies (MS-F203EXT/MS-F204EXT).

Design/Methods - In MS-F203EXT/MS-F204EXT, patients were treated chronically with 10 mg bid and were assessed in the clinic at 2, 14, 26 weeks and every 6 months thereafter. Patients treated with Fampridine-SR in the double-blind studies were categorized based on Double-Blind Timed Walk Responder (DBTWR) status. A DBTWR was defined as a patient whose WS was faster for at least 3 of the 4 double-blind efficacy visits compared with the maximum WS on any of 5 off-treatment visits.

Collective Analysis - Among 224 patients treated with Fampridine-SR in MS-F203, 197 patients entered the extension study and had at least one WS measurement. The improvement in WS observed in the double-blind study was lost after Fampridine-SR discontinuation, but returned at the first extension study efficacy visit. At 2.5 years from enrolment in MS-F203, the average change from baseline for DBTWRs remained above the original baseline while the non-DBTWRs had declined below the original baseline.

The analogous analysis conducted for MS-F204/MS-F204EXT yielded similar results at 1.2 years from enrolment in MS-F204 at data cutoff (of 113 patients treated with Fampridine-SR in MS-F204, 109 patients entered MS-F204EXT and had at least one WS measurement. No notable difference in tolerability was found between DBTWRs and non-DBTWRs, and no new safety issues were identified.

MS patients treated with Fampridine-SR showed consistent improvements compared to baseline in walking speed that were sustained for up to 2.5 years during open-label treatment. No new safety signals emerged.

### Example 4: Open-Label Extension Study (MSF-202EXT) to Evaluate the Safety, Tolerability and Activity of Oral Fampridine-SR in Subjects with Multiple Sclerosis

This is a long-term, multi-center, open-label study of continued treatment with Fampridine-SR. The target population consisted of patients throughout the United States and Canada who had been diagnosed with multiple sclerosis and who had participated in an Acorda or Elan sponsored clinical trial of Fampridine.

A total of 177 patients were enrolled. All patients had a Screening Visit, and, upon meeting the inclusion and exclusion criteria, returned within 14 days (Visit 0) to begin investigational drug dose escalation (up-titration). Patients were seen weekly for the first two weeks of investigational drug administration (Visits 1 and 2), while stabilizing on a dose. Patients were seen 4 weeks after Visit 2 (Visit 3) and again 8 weeks after Visit 3 (Visit 4). Thereafter, patients were seen every 12 weeks, with a telephone visit six weeks between each clinic visit. The visit interval was subsequently increased to every 26 weeks, with 2 telephone visits approximately 8 weeks and 16 weeks after each clinic visit. At the end of the study, patients returned for their next regularly scheduled 26-week visit (the Final Visit), and began a four-week follow-up period in which they take no investigational drug. They returned in 4 weeks for follow-up assessments (the Follow-up Visit).

Patients took one Fampridine-SR tablet every 12 hours throughout the study at approximately the same time each day. Patients could receive either Fampridine-SR 20 mg b.i.d. or 15 mg b.i.d. This was reduced to either Fampridine-SR 15 mg b.i.d. or 10 mg b.i.d. Finally, patients could receive only Fampridine-SR 10 mg b.i.d. (20 mg/day).

Functional assessments included:
- Timed 25 Foot Walk,
- Clinician Global Impression of Change (CGI),
- Subject Global Impression (SGI), and
- Expanded Disability Status Score (EDSS).

Safety evaluations were based on adverse event reporting, physical examination, clinical laboratory testing, 12-lead ECG, and (in a subset of patients) standard EEG testing. At each clinic visit, the Investigator was required to document whether or not continued participation in the study was in the patient's best interest. Patients terminating therapy were to undergo an Early Termination Visit.

Diagnosis and main criteria for inclusion - For inclusion into the trial, the patient must have been
- previously enrolled in a study sponsored by Acorda Therapeutics or Elan Corporation for multiple sclerosis and received either fampridine or placebo,
- to have had multiple sclerosis as determined by the Principal Investigator, and,
- male or female, must have been at least 18 years of age.

Any patient who was over the age of 70 must have been in good overall health in the judgment of the Investigator. Any female patient of childbearing potential, regardless of sexual activity, must have had a negative urine pregnancy test at the Screening Visit.

Test product, dose and mode of administration - The test product was Fampridine-SR 10 mg, 15 mg, and 20 mg tablets administered orally b.i.d. (every 12 hours).

Duration of treatment and estimated patient-year exposure - The 177 patients in the study have been exposed to Fampridine-SR 10 mg b.i.d. for approximately 2.6 years on average (range = 2 days, 4.5 years). One hundred seventy-five (175) of the 177 patients have been exposed to 15 mg b.i.d. for approximately 0.6 years on average (range = 2 days, 1 year); 9 of the 177 patients have been exposed to 20 mg b.i.d. for approximately 0.2 years on average (range = 15 days, 0.5 years). The estimated exposure with the clinical cutoff of 30 Nov 2008 for this cohort is approximately 567 patient-years.

Efficacy - The functional and subjective efficacy assessments included the Timed 25 Foot Walk, the Clinician Global Impression of Change (CGI), and the Subject Global Impression (SGI).

Safety - Safety evaluations included adverse event reporting, physical examination, clinical laboratory testing, vital signs, and 12-lead ECG.

Statistical methods - All computations were performed using SAS® Version 8.2 or above. All study data came from the updated pooled database using the 30 November clinical cut-off. Descriptive statistics (n, mean, standard deviation [SD], median, minimum and maximum) were calculated for continuous variables and ordinal variables with more than four categories. Frequencies and percentages were tabulated for all other categorical variables. Percentages were based on the total number of non-missing values. The safety population included all patients who received open-label investigational drug. This population was used in analyses of demographics and baseline characteristics data as well as in the analysis of the safety data. Patients could receive either Fampridine-SR 20 mg b.i.d. or 15 mg b.i.d. This was reduced to either Fampridine-SR 15 mg b.i.d. or 10 mg b.i.d. Finally, patients could receive only Fampridine-SR 10 mg b.i.d. Abbreviated safety analyses were conducted with respect to the following variables: treatment-emergent adverse events (TEAEs) and clinically significant: vital signs, laboratory assessments, and ECG parameters.

Patient Disposition - Enrolled: N = 177; Number discontinued = 84 (47.5%) (30 due to adverse event and 54 due to other reasons); Number active = 93 (52.5%)

Efficacy Results - The updated efficacy results from the ongoing open-label studies are provided in a combined Meta-analysis clinical study report (MS-F-EXT) which includes MS-F202EXT, MS-F203EXT, and MS-F204EXT. This Meta-analysis report examines signals of long-term efficacy of Fampridine-SR in multiple sclerosis patients.

Safety Results - The most common adverse events were urinary tract infection, falls, asthenia, multiple sclerosis relapse, insomnia, headache, muscle spasticity, fatigue, upper respiratory tract infection, dizziness, arthralgia, muscle spasms, and peripheral edema. In general, the adverse event pattern observed in this open-label extension study sub-population is similar to the adverse event pattern recorded for Fampridine-SR treated patients in the double-blind studies. Among the 177 patients who were treated, 93 remained ongoing as of 30 Nov 2008. Thirty patients were withdrawn from the study due to adverse events. Additionally, 2 patients were withdrawn for non-compliance, 32 withdrew consent, 3 were lost to follow-up, and 17 were withdrawn for 'Other' reasons. Fifty-eight patients experienced a serious adverse event at some point in the study, the most common being multiple sclerosis relapse. One patient experienced a complex partial seizure while on 10 mg b.i.d. Fampridine-SR, and two patients had generalized seizure (convulsion) at the dose of 15 mg b.i.d.

This study provides additional evidence of the long term efficacy and tolerability of Fampridine-SR in patients with multiple sclerosis. The retention of more than 50% of patients after nearly 5 years of study attests to the perceived benefit and safety of the treatment on the part of patients and investigating physicians.

Example 5: Interim Findings of Three Long-term Studies (MS-F202EXT, MS-F203EXT, MS-F204EXT)

Three long term studies (MS-F202EXT, MS-F203EXT, MS-F204EXT) are ongoing (as of this filing) multi-center, open-label extensions of continued treatment with 4-aminopyridine-SR for patients with clinically definite multiple sclerosis who participated in either the two Phase 3 studies (MSF-203, MSF-204) or in earlier Phase 2 (MS-F202) studies. The efficacy assessments are the Timed 25-Foot Walk, CGI and SGI at each visit, and the EDSS, assessed every two years.

Table 4 below, provides an overview of the studies and a short description of the results of each study. An assessment of pooled efficacy data of studies MS-F202, MS-F203 and MS-F204 is given in herein. In view of the purely descriptive nature of the analyses of the extension study data, these have been combined for all three extension studies at the end of this section.

**Table 4: Overall Design of 4-aminopyridine-SR Studies - MS-F202, MS-F203 and MS-F204 (MSWS-12 = The 12 Item Multiple Sclerosis Walking Scale; SGI = Subject Global Impression; CGI = Clinician Global Impression; LEMMT = Lower Extremity Manual Muscle Test):**

| | | | **Study Duration (weeks)** | | **Study Endpoints** | |
|---|---|---|---|---|---|---|
| **Study No., Protocol Name, Design** | **No. Patients** | **Dose, Regimen, Route** | **Double-Blind Period** | **Total Study** | **Primary** | **Secondary** |
| **MS-F202:** | 211 enrolled | FAM-SR Tab; 10, 15,20 mg; b.i.d.; Oral | 15 weeks | 20 weeks | ***Prospective primary endpoint:*** | ***Prospective secondary endpoints:*** |
| Double-Blind, Placebo-Controlled, 20-Week, Parallel Group Study to Evaluate Safety, Tolerability and Activity of Oral 4-aminopyridine-SR in Patients with Multiple Sclerosis | 206 Randomized | | | | percent change from baseline in average walking speed measured using the Timed 25-Foot Walk | a response criterion based on an average improvement of >20% in walking speed during the double-blind treatment period; average improvement in Lower Extremity Manual Muscle Test (LEMMT) score and 9-Hole Peg; Paced Auditory Serial Addition Test scores (both from the MS Functional Composite - MSFC); the MSFC combined score; spasticity assessment (Ashworth Score); Clinician's Global Impression of Change (CGI); Subject's Global Impression (SGI); the 12-Item MS Walking Scale (MSWS-12); and the Multiple Sclerosis Quality of Life Inventory (MSQLI). |
| ***Design:*** | (47, placebo; 52, 10 mg b.i.d. 50, 15 mg b.i.d. 57,20 mg b.i.d. 4-aminopyridine-SR) | | | | ***Post hoc responder analysis:*** | |
| Double-blind, randomized, placebo controlled, dose comparison study | | | | | Consistency of walking speed improvement (Timed Walk Response). A Responder was defined as a patient who had faster walking speed for at least three of four during the double-blind period as compared to the maximum speed among all five of the non double-blind (off) treatment visits. | |
| **MS-F203:** | 304 enrolled | FAM-SR Tab; 10 mg; b.i.d.; Oral | 14 weeks | 21 weeks | ***Prospective primary endpoint, as defined in theSPA:*** | ***Prospective, stepwise analysis of secondary endpoints:*** |
| Double-Blind, Placebo-Controlled, 21-week, Parallel Group Study to Evaluate Safety and Efficacy of Oral 4-aminopyridine-SR (10 mg b.i.d.) in Subjects with Multiple Sclerosis | 301 randomized | | | | Timed Walk Response, based on the Timed 25 Foot Walk. A Responder was defined as a patient who had faster walking speed for at least three of four during the double-blind period as compared to the maximum speed among the first five of the non double-blind (off) treatment visits. | • Change from baseline in LEMMT averaged over the double-blind treatment period and compared separately for Timed Walk Responders and Non-Responders |
| | (72, placebo; 229, 10 mg b.i.d. 4-aminopyridine-SR) | | | | | |
| ***Design:*** | | | | | | • Change from baseline in the Average Ashworth Score over the double-blind treatment period, and compared separately for Timed Walk Responders and Non-Responders. |
| Double-blind, randomized, placebo controlled study | | | | | | |
| | | | | | ***Additional requirements of the SPA:*** | |
| | | | | | Maintenance of effect defined as significantly greater improvement in walking speed at the last double-blind assessment for 4-aminopyridine-SR treated Timed Walk Responders compared to placebo-treated patients. | |
| | | | | | Validation of Timed Walk Response criterion-statistically significant greater improvement in MSWS-12 score for Timed Walk Responders compared to Timed Walk Non-Responders. | |
| **MS-F204:** | 240 enrolled | FAM-SR Tab; 10 mg; b.i.d.; Oral | 9 weeks | 14 weeks | ***Prospective primary endpoint, as defined in the SPA:*** | ***Prospective secondary endpoint:*** Average change from baseline in LEMMT during the eight-week, double-blind treatment period, comparing Timed Walk Responders and Timed Walk Non-Responders separately and sequentially against placebo-treated patients. |
| Double-Blind, Placebo-Controlled, Parallel Group Study to Evaluate Safety and Efficacy of Oral 4-aminopyidine-SR (10 mg b.i.d.) in Patients with Multiple Sclerosis | 239 randomized | | | | | |
| | (119, placebo; 120, 10 mg b.i.d. 4-aminopyridine-SR) | | | | Timed Walk Response, based on the Timed 25 Foot Walk. A Responder was defined as a patient who had faster walking speed for at least three of the first four visits during the double-blind period as compared to the maximum speed among all five of the non double-blind (off) treatment visits. | |
| ***Design:*** | | | | | | Pharmacokinetic data was to be collected at an additional fifth double-blind treatment visit (Visit 7) which was not part of the overall efficacy analysis. Additional assessments, including MSWS-12, SGI, CGI and Ashworth score, were collected for purpose of a pooled analysis with other studies and were not formal secondary endpoints. |
| Double-blind, randomized, placebo controlled study | | | | | | |

MS-F202EXT - As of the filing date, MS-F202EXT is an ongoing, long-term, multi-center, open-label extension study of continued treatment with 4-aminopyridine-SR for patients with clinically definite multiple sclerosis who previously participated in a study of 4-aminopyridine. As of July 31, 2008, there were 198 patients screened, 177 enrolled and approximately 98 remained active, based on clinical monitoring reports. Approximately 160 patients completed more than 6 months, 145 more than 1 year, and 90 more than 4 years in the study, as of July 31, 2008. An integrated report, MS-F-EXT, used data from all ongoing extension studies with a clinical cutoff date of July 31, 2008 to explore, the efficacy of 4-aminopyridine-SR with prolonged open-label treatment. The results are summarized herein.

MS-F203EXT - As of the filing date, MS-F203EXT is an ongoing long-term, multi-center, open-label extension study of continued treatment with 4-aminopyridine-SR for patients with clinically definite multiple sclerosis who participated in study MS-F203. As of July 31, 2008, there were 272 patients screened, 269 enrolled and approximately 196 remained active, based on clinical monitoring reports. Approximately 247 patients completed 6 months, 227 more than 1 year and 203 more than 2 years in the study as of July 31, 2008. An integrated report, MS-F-EXT, used data from all ongoing extension studies with a clinical cutoff date of July 31, 2008 to explore the efficacy of 4-aminopyridine-SR with prolonged open-label treatment. The results are summarized herein.

MS-F204EXT - As of the filing date, MS-F204EXT is an ongoing long-term, multi-center, open-label extension study of continued treatment with 4-aminopyridine-SR for patients with clinically definite multiple sclerosis who participated in study MS-F204. As of July 31, 2008, there were 219 patients screened, 214 enrolled and approximately 190 remained active, based on clinical monitoring reports. A total of 139 had completed 6 months in the study as of July 31, 2008. An integrated report, MS-F-EXT, used data from all ongoing extension studies with a clinical cutoff date of July 31, 2008 to explore the efficacy of 4-aminopyridine-SR with prolonged open-label treatment. The results are summarized herein.

*Evidence of Continued Efficacy in Long-term, Open-label Extension Studies -* A total of 756 patients participated in the three open label long term extension studies (MS-F202EXT, MS-F203EXT and MS-F204EXT) of which 546 patients completed 6 months, and 372 had completed more than 1 year, based on clinical monitoring reports, as of July 31, 2008. In the longest open study, MS-F202EXT, approximately 98 (55%) of the 177 recruited patients remained active in the study, the majority of them having completed more than 4 years of open-label treatment.

An integrated report, "MS-F-EXT", used Interim data from three ongoing extension studies (MS-F202 EXT, MS-F203 EXT, and MS-F204 EXT), with an interim clinical cutoff date of July 31, 2008 explored the longer-term efficacy of 4-aminopyridine-SR. The objectives, methodology, and key results are summarized below.

Objectives of MS-F-EXT: The purpose of the MS-F-EXT was to analyze the available efficacy data from ongoing, open-label, safety extension studies of 4-aminopyridine-SR in patients diagnosed with multiple sclerosis, with an interim data cut-off date of July 31, 2008.

Methodology of MS-F-EXT: The main focus of this report was to examine available data on walking speed, Subject, and Clinician Global Impressions for evidence of maintained response to treatment during the ongoing, open label extension phase of study.

The analysis of efficacy was based on all subjects who received at least one efficacy measurement in study MS-F202EXT, MS-F203EXT or MS-F204EXT and also participated in the parent double-blind study. For this purpose, an equivalent Timed Walk Response criterion was used for the extension study data, where an Extension Timed Walk Responder was defined as a patient showing walking speeds for the majority of on-treatment extension study visits that were faster than the fastest off-treatment walking speed recorded prior to the open-label treatment (i.e. speeds measured at all off treatment visits from the screening visit for the double-blind parent study through the screening visit for the extension study). Data were presented by study pair (parent and extension).

In order to characterize the efficacy of 4-aminopyridine-SR in treating patients with MS, the following analyses were performed:
1. Frequency of Extension Timed Walk Response in each of the extension studies.
2. The average percent changes in walking speed with respect to the double-blind baseline were presented in graphical form by Responder analysis groups for both parent and extension study visits.
3. In order to validate the clinical meaningfulness of the Extension Timed Walk Response criterion, the average of the Subject Global Impression (SGI) scores and the average of the Clinician Global Impression (CGI) scores during each extension study were compared between Extension Timed Walk Responders and Non-Responders.
4. In addition, Extension Timed Walk Response rates within successive years of treatment were summarized by frequency tables.
5. Changes in the Expanded Disability Status Scale (EDSS) scores were compared between the Extension Timed Walk Responder groups, where available (evaluated only every 2 years).
6. An alpha level of 0.05 was used in the analysis. Correction for multiple tests was not used.

Observations and Findings Following Chronic/Prolonged/Extended Administration of 4-Aminopyridine:

In study MS-F202EXT, a total of 21 (15.7%) of patients were classified as Extension Timed Walk Responders. A total of 11(25.6%) of the 4-aminopyridine-treated Timed Walk Responders from the parent study (MS-F202) continued to be Extension Timed Walk Responders; in addition, 6 (9.5%) of the 4-aminopyridine-treated Timed Walk Non-Responders from the parent study became Extension Timed Walk Responders and 4(14.3%) of the placebo-treated patients from the parent study qualified as Extension Timed Walk Responders. The percentages of 4-aminopyridine double-blind Responders who continued to be Extension Timed Walk Responders in years 1, 2 and 3 of the extension study were 25.6%, 23.1% and 22.2%, respectively. For the double-blind Timed Walk Non-Responders these numbers were 11.1%, 5.2% and 6.1%, respectively and for the placebo treated patients 17.9%, 4.6% and 5.3%, respectively.

In study MS-F203EXT, a total of 66 (24.9%) of patients were classified as Extension Timed Walk Responders. Among them, 29(41.4%) of the 4-aminopyridine-Treated Timed Walk Responders from the parent study (MS-F203) continued to be Extension Timed Walk Responder; in addition, 25(19.7%) of the 4-aminopyridine-treated Timed Walk Non-Responders from the parent study became Extension Timed Walk Responders and 12(17.7%) of the placebo-treated patients from the parent study qualified as Extension Timed Walk Responders. The year one and year 2 response rates were 42.9% and 36.1%, respectively for 4-aminopyridine double-blind Responders; 19.7% and 17.5%, respectively for the 4-aminopyridine double-blind Non-Responders; and 16.2% and 20.8%, respectively for the placebo treated patients.

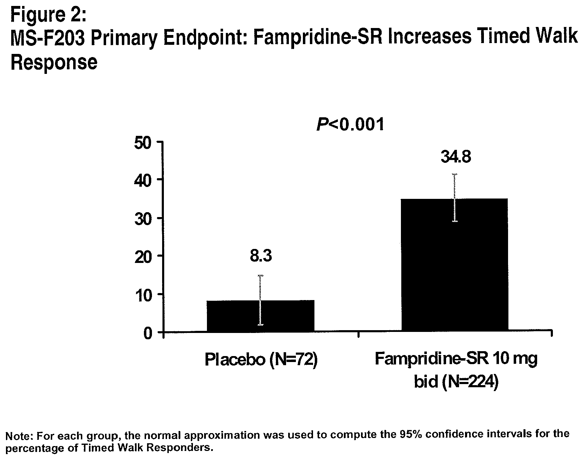

The average percent change from baseline walking speed for the Extension Timed Walk Responders and Extension Timed Walk Non-Responders was graphed for all patients in MS-F203EXT in Figure 2, below, for the period of both the parent study and the first two years of the extension study. The mean walking speed for the Extension Timed Walk Responder group at each extension study visit was slightly more than 30% faster than the baseline walking speed from the double blind study, for the first year of the extension study. The Extension Timed Walk Non-Responders showed little change from baseline in mean walking speed over the course of the year, except for a slight increase after the first two weeks on drug (Visit 1) and a slight decrease in the mean at one year (Visit 4). Some decline in mean walking speed improvement was seen for the Timed Walk Responders in the second year of the extension study, so that the improvement over the original baseline was only slightly more than 20% at Visit 6. Also by the end of the second year, the Timed Walk Non-Responders had declined in walking speed by approximately 8% from the original double-blind study baseline, consistent with or based on the progressive nature of the underlying disease.

In study MS-F204EXT, a total of 105 (49.3%) of patients were classified as Extension Timed Walk Responders. Among them, 35 (71.4%) of the 4-aminopyridine-Treated Timed Walk Responders from the parent study (MS-F204) continued to be Extension Timed Walk Responders; in addition, 18 (30.0%) of the 4-aminopyridine-treated Timed Walk Non-Responders from the parent study became Extension Timed Walk Responders and 52 (50.0%) of the placebo-treated patients from the parent study qualified as Extension Timed Walk Responders. The improvements among patients assessed in the study occurs over periods of at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years of treatment.

Therefore, consistent improvement in walking speed was seen in a significant proportion of patients in the long term extension studies, MS-F202EXT, MS-F203EXT and MS-F204EXT using the primary endpoint, Timed Walk Response (which was used in the double-blind, controlled parent studies, MS-F202, MS-F203 and MS-F204). This improvement among Extension Timed Walk Responders was stable over at least the first two years of treatment. The improvement(s) among patients addressed in these studies occurs/occur over periods of at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years of treatment.

Overall, the improvement(s) among patients addressed in these studies occurs/occur over periods of at least or more than: 8, 9, 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years of treatment. These findings further support the clinical meaningfulness of the improvements seen in the double-blind and extension studies as well as the validity of the criterion used to identify this ambulatory response to treatment.

Table 5 below provides an overview of primary and secondary efficacy variables in the studies MS-F201, MS-F202, MS-F203 and MS-F204.

**Table 5: Efficacy and Health Outcome Measures used in Placebo-Controlled Efficacy Studies:**

| | **Study** | | | |
|---|---|---|---|---|
| **Measures** | **MS-F203** | **MS-F204** | **MS-F201** | **MS-202** |
| ***Primary Variable*** | | | | |
| Timed-Walk Response (at least 3 on-treatment visit T25FW speeds faster than the fastest off-treatment speed) | X | X | | X (retrospectively) |

| ***Secondary Variables*** | | | | |
|---|---|---|---|---|
| T25FW speed at each visit | X | X | X | X |
| Lower Extremity Muscle Testing (LEMMT) | X | X | X | X |
| Spasticity assessment (Ashworth score) | X | X | | X |
| 12-Item MS Walking Scale (MSWS-12) | X | X | | X |
| Clinician Global Impression of Change (CGI) | X | X | X | X |
| Subject's Global Impression (SGI) | X | X | X | X |
| Response of >20% avg improvement in walking speed | | | | X |
| MS Functional Composite Score | | | X | X |
| Brief Fatigue Inventory (BFI) | | | X | |
| Modified Fatigue Impact Scale (MFIS) | | | X | |
| MS Quality of Life Inventory (MSQLI) | | | | X |

A non-exhaustive list of embodiments of the invention is provided in the following numbered clauses:
1. A method for use of 4-aminopyridine over chronic periods such as 1, 2, 3, 4, 5, 6, ,7, 8, 9, 10, 11, 12, 13, 14, 15, 16, 17, 18 or more than 18 months to achieve therapeutic benefit in patients with demyelinating conditions such as MS as set forth herein.
2. A method for use of 4-aminopyridine over chronic periods such as 1, 2, 3, 4, 5, 6, ,7, 8, 9, 10, 11, 12, 13, 14, 15, 16, 17, 18 or more than 18 years to achieve therapeutic benefit in patients with demyelinating conditions such as MS as set forth herein.
3. The method of clause 1 or 2, wherein walking speed is improved.
4. A method for durable treatment of a patient having multiple sclerosis comprising administering to the patient an effective amount of 4-aminopyridine at least once daily for at least 12 months.
5. The method of clause 4, further comprising monitoring patient blood levels of 4-aminopyridine and adjusting the dosage of 4-aminopyridine to maintain patient blood levels of 4-aminopyridine within a therapeutic range.

## Claims

1. A sustained release 4-aminopyridine composition for use in achieving a sustained improvement in walking speed in a multiple sclerosis patient over a chronic period of at least 12 months.

2. The composition as claimed in claim 1, wherein the chronic period is at least 18 months or at least two years.

3. The composition as claimed in claim 1 or claim 2, wherein the composition is administered twice daily in a dose of 10 mg 4-aminopyridine.

4. Use of 4-aminopyridine in the manufacture of a sustained release medicament for achieving a sustained improvement in walking speed in a multiple sclerosis patient over a chronic period of at least 12 months.

5. The use as claimed in claim 4, further comprising any of the feature(s) of claims 2 or 3.
